# EUROPÄISCHE PATENTSCHRIFT

(11) **EP 0 815 078 B1**
(45) Veröffentlichungstag und Bekanntmachung des Hinweises auf die Patenterteilung: **02.04.2003**
(21) Anmeldenummer: 96919688.0
(22) Anmeldetag: 07.05.1996
(51) Int. Cl.: C07D 201/08, C07D 223/12

(54) **VERFAHREN ZUR HERSTELLUNG VON CAPROLACTAM**
PROCESS FOR PRODUCING CAPROLACTAM
PROCEDE DE PREPARATION DE CAPROLACTAME

(30) Priorität: 18.05.1995 DE 19517823
(43) Veröffentlichungstag der Anmeldung: 07.01.1998
(73) Patentinhaber: BASF AKTIENGESELLSCHAFT, 67056 Ludwigshafen (DE)
(72) Erfinder: FUCHS, Eberhard, D-67227 Frankenthal (DE); MELDER, Johann-Peter, D-67141 Neuhofen (DE); SCHNURR, Werner, D-67273 Herxheim (DE); FISCHER, Rolf, D-69121 Heidelberg (DE)
(86) Internationale Anmeldenummer: EP9601892
(87) Internationale Veröffentlichungsnummer: WO96036601

(56) Entgegenhaltungen:
- DE-A- 2 542 396
- DE-A- 4 339 648
- DE-C- 749 469
- US-A- 2 301 964
- US-A- 2 357 484
- US-A- 5 162 567

## Beschreibung

Die vorliegende Erfindung betrifft ein verbessertes Verfahren zur Herstellung von Caprolactam durch Umsetzung von 6-Aminocapronitril mit Wasser bei erhöhter Temperatur in Gegenwart von Titandioxid als heterogenem Katalysator.

Durch Erhitzen von 6-Aminocapronitril als auch bei Raumtemperatur, beispielsweise beim Lagern von 6-Aminocapronitril, entsteht ein braunes Tetrahydroazepin-Derivat (THA-Derivat I) der Formel

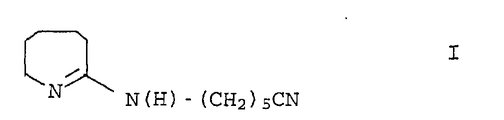

Unter THA-Derivat I sei auch dessen tautomere Form 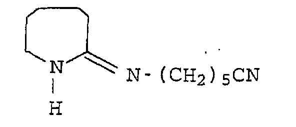 mit eingeschlossen.

Aus DE-C-74 94 695 ist die Herstellung von TMA-Derivat (I) und dessen Verwendung als Ausgangstoff für die Herstellung plastischer Massen generisch offenbart; Caprolactam oder Polycaprolactam sind nicht erwähnt.

DE-A-43 39 648 betrifft die Herstellung cyclischer Lactame einschließlich Caprolactam in Anwesenheit von Wasser in der Flüssigphase ausgehend von 6-Aminocapronitril in Gegenwart eines heterogenen Katalysators.

US-A-2 357 484 offenbart die Herstellung cyclischer Lactame, einschließlich Caprolactam in der Gegenphase in Anwesenheit von Wasser ausgehend von 6-Aminocapronitril.

US-A-2 301 964 betrifft die Herstellung cyclischer Lactame einschließlich Caprolactam in Anwesenheit von Wasser in der Flüssigphase ausgehend von 6-Aminocapronitril in Abwesenheit eines Katalysators.

Die EP-A 497,333 beschreibt die Direktpolymerisation von Polycaprolactam ausgehend von 6-Aminocapronitril. Das zu lösende Problem bei dem genannten Verfahren war die Entfernung von Tetrahydroazepin ("THA") vor dem Polymerisationsschritt, da Tetrahydroazepin zu einer unerwünschten Verfärbung des Polymeren führt, wenn man Caprolactam in Gegenwart von Tetrahydroazepin polymerisiert. In der EP-A 497,333 wird als Lösung die Behandlung mit einer basischen Verbindung wie ein Alkalimetallhydroxid oder ein Alkalimetallalkoxid vorgeschlagen. Nach der Behandlung kann man 6-Aminocapronitril bequem aus dem Reaktionsgemisch durch Destillation abtrennen, was ohne eine entsprechende Behandlung nicht möglich ist.

In der EP-A 502,439 wird das Problem der Entfernung von THA in Gegenwart von 6-Aminocapronitril durch eine Behandlung mit Natriumborhydrid gelöst. Auch hier kann man nach dem Behandlungsschritt 6-Aminocapronitril gut aus dem Reaktionsgemisch durch Destillation abtrennen.

Die DE-B-25 42 396 und DE-B-25 42 397 beschreiben die Umsetzung von gamma-Aminobutyronitril zu einem Gemisch, enthaltend 2-(N-gammacyanopropyl)-amino-delta¹-pyrrolin ("CAP") und 2-Aminodelta¹-pyrrolin ("AP"), sowie die weitere Hydrolyse des isolierten CAP zu Pyrrolidon-2 in Abwesenheit von Katalysatoren. Beide DE-Auslegeschriften geben keinerlei Hinweise darauf, ob sich das entsprechende THA-Derivat I in Gegenwart von heterogenen Katalysatoren in flüssiger Phase analog zu Caprolactam umsetzen läßt. Ferner wird in den genannten Druckschriften CAP zunächst als Reinsubstanz isoliert, bevor CAP hydrolysiert wird. Es war daher zu erwarten, daß es bei der Verwendung von THA-Derivat I enthaltenden Mischungen, vermehrt zu unerwünschten Nebenprodukten kommt. Des weiteren ist bekannt, daß sich Fünfringe leichter bilden als siebengliedrige Ringe (siehe Römpp Chemie Lexikon, 9. Auflage, Hrsg. Falbe und Regitz, Georg Thieme Verlag, New York). Insgesamt und aufgrund der Erfahrungen mit THA war daher zu erwarten, daß das THA-Derivat I sowohl bei der Cyclisierung von 6-Aminocapronitril zu verfärbtem Caprolactam als auch bei der direkten Umsetzung von 6-Aminocapronitril zu Polycaprolactam zu verfärbtem Polycaprolactam führen würde, falls man es nicht bereits vor der Cyclisierung und vor dem Polymerisationsschritt abtrennt.

Ferner war damit zu rechnen, daß das THA-Derivat I die Standzeit des bei der Polymerisation eingesetzten Katalysators verringert, da aus der US-A-5,162,567 bekannt war, daß beim Erhitzen von THA Hochsieder, d.h. Substanzen oder Substanzgemische entstehen, die höher als 6-Aminocapronitril sieden (und 6-Aminocapronitril hierdurch entsprechend auch gut abgetrennt werden kann). Hochsieder neigen jedoch zur Bildung polymerer oder oligomerer Zersetzungsprodukte, die sich auf der Oberfläche von Katalysatoren ablagern können und hierdurch sowohl Standzeit als auch die Aktivität der eingesetzten Katalysatoren verringern.

Die Aufgabe der vorliegenden Erfindung war daher die Bereitstellung eines Verfahrens zur Cyclisierung von 6-Aminocapronitril zu Caprolactam, bei dem das THA-Derivat I weder die Standzeit noch die Aktivität des bei der Cyclisierung eingesetzten Katalysators verringert, noch zu einem Caprolactam-enthaltenden Reaktionsgemisch führt, dessen UV-Kennzahl gleich oder höher ist als vor dem Cyclisierungsschritt. Bevorzugt sollte die UV-Kennzahl nach dem Cyclisierungsschritt in Abhängigkeit vom Gehalt an THA-Derivat I vor der Cyclisierung kleiner sein als vor dem Cyclisierungsschritt. Des weiteren sollte das bei der Direktpolymerisation von 6-Aminocapronitril gegebenenfalls im Reaktionsgemisch vorhandene THA-Derivat I entweder leicht abgetrennt oder die Reaktionsführung so gewählt werden können, daß das THA-Derivat I eliminiert wird.

Demgemäß wurde ein Verfahren zur Herstellung von Caprolactam durch Umsetzung von 6-Aminocapronitril mit Wasser bei erhöhter Temperatur in Gegenwart von Titandioxid als heterogenem Katalysator gefunden, indem man eine Ausgangsmischung aus 6-Aminocapronitril und dem Tetrahydroazepin-Derivat der Formel 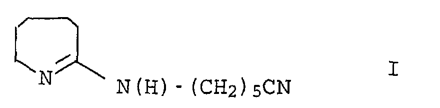 in Form einer 1 bis 50 gew.-%igen Lösung in Wasser/organischen Lösungsmittelgemischen einsetzt wobei der Gehalt des Tetrahydroazipin-Derivats (I) in bezug auf das 6-Aminocapronitril in der Ausgangsmischung 0,01 bis 95 Gew.-% beträgt, und die Umsetzung in flüssiger Phase bei Temperaturen im Bereich von 160 bis 280°C und einer Verweilzeit von 1 bis 120 min durchführt.

Die Umsetzung wird erfindungsgemäß in flüssiger Phase in Gegenwart von Titandioxid als heterogenem Katalysator bei Temperaturen von 160 bis 280°C, durchgeführt; der Druck liegt im allgemeinen im Bereich von 1 bis 250 bar, vorzugsweise von 5 bis 150 bar, wobei darauf zu achten ist, daß das Reaktionsgemisch unter den angewandten Bedingungen zum überwiegenden Teil, d.h. ohne den in fester Phase vorliegenden Katalysator, flüssig ist. Die Verweilzeiten liegen erfindungsgemäß im Bereich von 1 bis 120, vorzugsweise 1 bis 90 und insbesondere 1 bis 60 min. In einigen Fällen haben sich Verweilzeiten von 1 bis 10 min als völlig ausreichend erwiesen.

Pro mol THA-Derivat I werden im allgemeinen mindestens 0,01 mol, vorzugsweise 0,1 bis 20 und insbesondere 1 bis 5 mol Wasser eingesetzt.

Erfindungsgemäß wird das THA-Derivat I in Form einer 1 bis 50 gew.-%igen, insbesondere 5 bis 50 gew.-%igen, besonders vorzugsweise 5 bis 30 gew.-%igen Lösung in Wasser/Lösungsmittelgemischen eingesetzt. Als Lösungsmittel seien beispielhaft Alkanole wie Methanol, Ethanol, n- und i-Propanol, n-, i- und t-Butanol und Polyole wie Diethylenglykol und Tetraethylenglykol, Kohlenwasserstoffe wie Petrolether, Benzol, Toluol, Xylol, Lactame wie Pyrrolidon oder Caprolactam oder alkylsubstituierte Lactame wie N-Methylpyrrolidon, N-Methylcaprolactam oder N-Ethylcaprolactam sowie Carbonsäureester, vorzugsweise von Carbonsäuren mit 1 bis 8 C-Atomen genannt. Auch Ammoniak kann bei der Reaktion anwesend sein. Selbstverständlich können auch Mischungen organischer Lösungsmittel Anwendung finden. Mischungen aus Wasser und Alkanolen im Gewichtsverhältnis Wasser/Alkanol 1 bis 75 zu 25 bis 99, vorzugsweise 1 bis 50 zu 50 bis 99, haben sich in einigen Fällen als besonders vorteilhaft herausgestellt.

Der Gehalt des THA-Derivates I in bezug auf das 6-Aminocapronitril in der Ausgangsmischung liegt bei 0,01 bis 95 Gew.-%, insbesondere 0,1 bis 50 Gew.-%, besonders bevorzugt 0,5 bis 20 Gew.-%.

Üblicherweise weist die Ausgangsmischung, in Abhängigkeit des Gehaltes an THA-Derivat I, eine UV-Kennzahl (angegeben als Summe aller Extinktionen gemessen an einer 10 gew.-%igen Lösung in Ethanol bei Wellenlängen von 280 bis 400 nm, bezogen auf eine Küvettenlänge von 5 cm) im Bereich von 5 bis 40000 auf.

Die Ausgangsmischung ist erhältlich durch Erhitzen von 6-Aminocapronitril mit oder ohne Lösungsmittel. Die Temperatur kann nach bisherigen Beobachtungen 20 bis 280°C, insbesondere 50 bis 250°C, besonders bevorzugt 100 bis 230°C, betragen. Die Reaktionszeiten liegen üblicherweise im Bereich von 10 Minuten bis 20 Stunden. Dabei sind, wie zu erwarten, bei höheren Temperaturen kürzere Reaktionszeiten einzuhalten. Die Umsetzung kann bei Drücken im Bereich von 100 kPa bis 25 MPa, bevorzugt von 500 kPa bis 20 MPa, durchgeführt werden. Des weiteren kann es vorteilhaft sein, die Umsetzung in Gegenwart von sauren homogenen oder heterogenen Katalysatoren wie Mineralsäure, Carbonsäuren, Sulfonsäuren, Titandioxid, Aluminiumoxid, sauren Ionenaustauschern oder Lewissäuren durchzuführen.

Gewünschtenfalls kann man reines THA-Derivat I beispielsweise durch Destillation von unumgesetztem 6-Aminocapronitril, Lösungsmitteln und gegebenenfalls Nebenprodukten gewinnen.

Als heterogener Katalysator kann Titandioxid beispielsweise amorph, als Anatas und/oder Rutil, oder Mischungen davon verwendet werden.

Die Katalysatoren können je nach der Zusammensetzung des Katalysators als Vollkontakt oder Trägerkatalysator verwendet werden. So kann Titandioxid beispielsweise als Titandioxid-Strang oder als auf einen Träger in dünner Schicht aufgebrachtes Titandioxid eingesetzt werden. Zum Aufbringen von TiO₂ auf einen Träger wie Siliciumdioxid, Aluminiumoxid oder Zirkondioxid sind alle in der Literatur beschriebenen Methoden verwendbar. So kann eine dünne TiO₂-Schicht durch Hydrolyse von Ti-Organylen wie Ti-Isopropylat oder Ti-Butylat, oder durch Hydrolyse von TiCl₄ oder anderen anorganischen Ti-haltigen Verbindungen aufgebracht werden. Auch Titandioxid-haltige Sole sind verwendbar.

In einer weiteren bevorzugten Ausführungsform führt man die Umsetzung in einem Festbettreaktor durch. Bei der Festbettfahrweise werden üblicherweise Tabletten oder Stränge mit Durchmessern zwischen 1 und 10 mm verwendet. Prinzipiell kann man jedoch auch die Umsetzung in Suspensionsfahrweise durchführen.

Reines Titandioxid kann durch Fällung aus wäßrigen Lösungen hergestellt worden sein, z.B. nach dem Sulfatprozeß oder durch andere Verfahren wie die pyrogene Herstellung von feinen Titandioxid- Pulvern, die käuflich zu erhalten sind.

Zur Herstellung von Trägerkatalysatoren bieten sich die üblichen Methoden an. So kann Titandioxid in Form seiner Sole durch einfaches Tränken auf dem Träger aufgebracht werden. Durch Trocknen und Calzinieren werden die flüchtigen Bestandteile des Sols üblicherweise aus dem Katalysator entfernt. Solche Sole sind für Titandioxid käuflich erhältlich.

Eine weitere Möglichkeit zum Aufbringen von Schichten der aktiven Oxide besteht in der Hydrolyse oder Pyrolyse von organischen oder anorganischen Verbindungen. So kann ein keramischer Träger mit Titandioxid durch Hydrolyse von Titan-Isopropylat oder anderen Ti-Alkoxiden in dünner Schicht belegt werden. Weitere geeignete Verbindung ist unter anderen TiCl₄. Geeignete Träger sind Pulver, Stränge oder

Tabletten der genannten Oxide selbst oder anderer stabiler Oxide wie Siliciumdioxid. Die verwendeten Träger können zur Verbesserung des Stofftransports makroporös ausgestaltet sein.

In einer weiteren besonders bevorzugten Ausführungsform setzt man Titandioxid als Katalysator ein, das einen Anatas-Gehalt im Bereich von 100 bis 5, bevorzugt von 99 bis 10 Gew.-% und einen Rutil-Gehalt im Bereich von 0 bis 95, bevorzugt von 1 bis 90 Gew.-%, bezogen auf den Gesamtgehalt an Titandioxid, aufweist.

Der Vorteil des erfindungsgemäßen Verfahrens besteht darin, einen Weg gefunden zu haben, in dem man THA-Derivat I enthaltende Reaktionsmischungen mit 6-Aminocapronitril ohne Schwierigkeiten zu Caprolactam verarbeiten kann. Die so erhaltenen Produkte bzw. Produktmischungen enthalten kein störendes THA-Derivat I mehr. Somit erübrigen sich weitere Verfahrensschritte und der Einsatz von zusätzlichen Mitteln verglichen mit der Entfernung von THA in entsprechenden Reaktionsmischungen.

Unter Umständen kann es sogar vorteilhaft sein, 6-Aminocapronitril durch eine Vorerhitzung auf Temperaturen von 20 bis 280°C ganz oder teilweise in das THA-Derivat I umzuwandeln und das so erhaltene Gemisch aus THA-Derivat I und 6-Aminocapronitril für die Cyclisierung an Titandioxid zu verwenden.

### Beispiele

### Beispiel 1:

400 g 6-Aminocapronitril (ACN) wurden 8 h auf 200°C erhitzt. Durch Destillation erhielt man als zweite Fraktion bei 0,1 mbar und 140°C 40 g THA-Derivat I (Ausbeute 10 %) als reine Verbindung. Die Charakterisierung erfolgte mittels NMR-Spektroskopie:
¹H-NMR (250 MHz, DMSO-d₆, TMS, ppm):
4,2 (s, breit, 1 H); 3,2 (m, 2 H); 2,9 (t, 2H); 2,45 (t, 2H); 2,25 (m, 2H); 1,7 - 1,1 (m, 12 H).
¹³C-NMR (62,9 MHz, DMSO-d₆, TMS, ppm):
163,3 s; 120,6 s; 47,0 t; 41,6 t; 32,9 t; 30,6 t; 29,7 t; 28,4 t; 26,0 t; 25,6 t; 24,8 t; 16,2 t.

### Beispiel 2:

Eine 10 gew.-%ige ethanolische Lösung von THA-Derivat I wurde zusammen mit 2 mol Wasser (entsprechend 3,2 Gew.-% der Gesamtlösung) mit 70 ml/h durch einen mit Titandioxid gefüllten Rohrreaktor (Durchmesser 6 mm; Länge 800 ml) gepumpt. Die Reaktortemperatur betrug 230°C, der Druck 80 bar. Man erhielt stündlich eine 9,7%ige ethanolische Caprolactamlösung. Weiterhin erhielt die Lösung 0,8 Gew.-% rückführbaren 6-Aminocapronsäureethylester sowie ebenfalls 0,2 Gew.-% rückführbares 6-Aminocapronsäurenitril. Die Caprolactamausbeute betrug 80 %, die Selektivität inklusive der rückführbaren Verbindungen 95 %.

### Beispiel 3:

Eine 10 gew.-%ige ethanolische Lösung, bestehend aus 95 Gew.-% ACN und 5 Gew.-% THA-Derivat I, wurde zusammen mit 2 mol Wasser (entsprechend 3,2 Gew.-% der Gesamtlösung) mit 70 ml/h durch einen mit Titandioxid gefüllten Rohrreaktor (Durchmesser 6 mm; Länge 800 ml) gepumpt. Die Reaktortemperatur betrug 230°C, der Druck 80 bar. Man erhielt stündlich eine 9,1 gew.-%ige ethanolische Caprolactamlösung. Weiterhin enthielt die Lösung 0,4 Gew.-% rückführbaren 6-Aminocapronsäureethylester sowie ebenfalls 0,1 Gew.-% rückführbares 6-Aminocapronsäurenitril. Die Caprolactamausbeute betrug 91 %, die Selektivität inklusive der rückführbaren Verbindungen 95 %.

### Beispiel 4:

Eine 10 gew.-%ige ethanolische Lösung, bestehend aus 99 Gew.-% ACN und 1 Gew.-% THA-Derivat I, wurde zusammen mit 2 mol Wasser (entsprechend 3,2 Gew.-% der Gesamtlösung) mit 70 ml/h durch einen mit Titandioxid gefüllten Rohrreaktor (Durchmesser 6 mm; Länge 800 ml) gepumpt. Die Reaktortemperatur betrug 230°C, der Druck 80 bar. Man erhielt stündlich eine 9,0 gew.-%ige ethanolische Caprolactamlösung. Weiterhin enthielt die Lösung 0,4 Gew.-% rückführbaren 6-Aminocapronsäureethylester sowie ebenfalls 0,1 Gew.-% rückführbares 6-Aminocapronsäurenitril. Die Caprolactamausbeute betrug 90 %, die Selektivität, inklusive der rückführbaren Verbindungen, 95 %.

## Patentansprüche

1. Verfahren zur Herstellung von Caprolactam durch Umsetzung von 6-Aminocapronitril mit Wasser bei erhöhter Temperatur in Gegenwart von Titandioxid als heterogenem Katalysator, **dadurch gekennzeichnet, daß** man eine Ausgangsmischung aus 6-Aminocapronitril und dem Tetrahydroazepin-Derivat der Formel 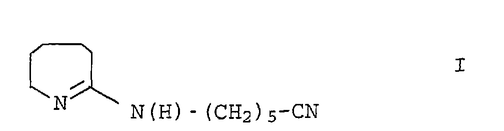 in Form einer 1 bis 50 gew.-%igen Lösung in Wasser/organischen Lösungsmittelgemischen einsetzt, wobei der Gehalt des Tetrahydroazepin-Derivates (I) in bezug auf das 6-Aminocapronitril in der Ausgangsmischung 0,01 bis 95 Gew.-% beträgt, und die Umsetzung in flüssiger Phase bei Temperaturen im Bereich von 160 bis 280°C und einer Verweilzeit von 1 bis 120 min durchführt.

2. Verfahren gemäß Anspruch 1, **dadurch gekennzeichnet, daß** man die Umsetzung in einem Festbettreaktor durchführt.

3. Verfahren gemäß Anspruch 1 oder 2, **dadurch gekennzeichnet, daß** man Titandioxid als Katalysator verwendet, das einen Anatas-Gehalt im Bereich von 5 bis 100 Gew.-% und einen Rutil-Gehalt im Bereich von 0 bis 95 Gew.-%, bezogen auf den Gesamtgehalt an Titandioxid, aufweist.

4. Verfahren gemäß den Ansprüchen 1 bis 3, **dadurch gekennzeichnet, daß** die Ausgangsmischung eine UV-Kennzahl (angegeben als Summe aller Extinktionen gemessen an einer 10 gew.-%igen Lösung in Ethanol bei Wellenlängen von 280 bis 400 nm, bezogen auf eine Küvettenlänge von 5 cm) im Bereich von 5 bis 40000 aufweist.

## Claims

1. A process for preparing caprolactam by reacting 6-aminocapronitrile with water at elevated temperature in the presence of titanium dioxide as a heterogeneous catalyst, which comprises using a starting mixture of 6-aminocapronitrile and the tetrahydroazepine derivative of the formula 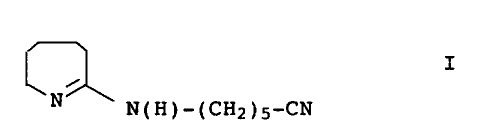 in the form of a 1 - 50% by weight solution in water/organic solvent mixtures, the level of the tetrahydroazepine derivative (I) based on the 6-aminocapronitrile in the starting mixture being 0.01 - 95% by weight, and conducting the reaction in liquid phase at 160 - 280°C using a residence time from 1 to 120 min.

2. A process as claimed in claim 1, wherein the reaction is carried out in a fixed bed reactor.

3. A process as claimed in claim 1 or 2, wherein the catalyst used comprises titanium dioxide having an anatase content within the range from 5 to 100% by weight and a rutile content within the range from 0 to 95% by weight, based on the total amount of titanium dioxide.

4. A process as claimed in any of claims 1 to 3, wherein the starting mixture has a UV number (sum of all absorbances of a 10% by weight solution in ethanol at wavelengths from 280 to 400 nm, based on a pathlength of 5 cm) within the range from 5 to 40,000.

## Revendications

1. Procédé de préparation de caprolactame par réaction de 6-aminocapronitrile avec de l'eau à température élevée, en présence de dioxyde de titane comme catalyseur hétérogène, **caractérisé en ce qu'**on met en oeuvre un mélange de départ à base de 6-aminocapronitrile et du dérivé de tétrahydroazépine de la formule : 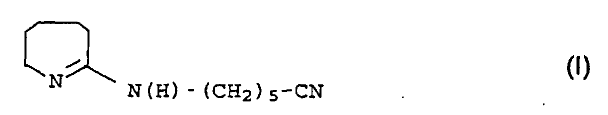 sous la forme d'une solution à 1 jusqu'à 50% en poids dans des mélanges d'eau/solvant organique, la teneur du dérivé de tétrahydroazépine (I) par rapport au 6-aminocapronitrile étant de 0,01 à 95% en poids dans le mélange de départ, et **en ce qu'**on effectue la réaction en phase liquide à des températures de l'ordre de 160 à 280°C et pendant une durée de séjour de 1 à 120 minutes.

2. Procédé suivant la revendication 1, **caractérisé en ce qu'**on effectue la réaction dans un réacteur à lit fixe.

3. Procédé suivant l'une des revendications 1 et 2, **caractérisé en ce qu'**on utilise, comme catalyseur, du dioxyde de titane qui présente une teneur en anatase de l'ordre de 5 à 100% en poids et une teneur en rutile de l'ordre de 0 à 95% en poids, par rapport à la teneur totale en dioxyde de titane.

4. Procédé suivant l'une des revendications 1 à 3, **caractérisé en ce que** le mélange de départ présente une constante U.V. (indiquée comme la somme de toutes les extinctions mesurées sur une solution à 10% en poids dans de l'éthanol à des longueurs d'onde de 280 à 400 nm, par rapport à une longueur de cuvette de 5 cm) de l'ordre de 5 à 40.000.
